# FASCICULE DE BREVET EUROPEEN

(11) **EP 2 961 379 B1**
(45) Date de publication et mention de la délivrance du brevet: **27.12.2017**
(21) Numéro de dépôt: 14713171.8
(22) Date de dépôt: 24.02.2014
(51) Int. Cl.: A61K 8/67, A61Q 19/08, A61K 8/97, A61K 8/99

(54) **COMPOSITION COSMÉTIQUE CONTENANT UN EXTRAIT D'ALGUE BRUNE, UN EXTRAIT DE LEVURE ET D'ACIDE ASCORBIQUE**
KOSMETIKZUSAMMENSETZUNG MIT EINEM BRAUNALGENEXTRAKT, EINEM HEFEEXTRAKT UND ASCORBINSÄURE
COSMETIC COMPOSITION CONTAINING A BROWN ALGA EXTRACT, A YEAST EXTRACT AND ASCORBIC ACID

(30) Priorité: 28.02.2013 FR 1351809
(43) Date de publication de la demande: 06.01.2016
(73) Titulaire: LvmH Recherche, 45800 Saint-Jean De Braye (FR)
(72) Inventeur: FRANCHI, Jocelyne, F-45140 Saint Jean De La Ruelle (FR); RENIMEL, Isabelle, F-45470 Trainou (FR); NEVEU, Michèle, F-45000 Orleans (FR); GORZELANCZYK, Valérie, F-45110 Chateauneuf Sur Loire (FR); MANIGUET, Sabrina, F-45470 Trainou (FR)
(74) Mandataire: Mena, Sandra
(86) Numéro de dépôt international: PCT/FR2014/050373
(87) Numéro de publication internationale: WO 2014/131971

(56) Documents cités:
- EP-A1- 1 938 789
- EP-A2- 1 514 537
- WO-A1-2010/145008
- WO-A2-2011/116216
- DATABASE GNPD [Online] MINTEL; septembre 2009 (2009-09), "Dimple Vanisher", XP055004233, Database accession no. 1146830
- DATABASE GNPD [Online] MINTEL; février 2007 (2007-02), "Skin Perfecting Serum", XP002715412, Database accession no. 663094

## Description

L'invention a pour objet une composition de soin cosmétique contenant une nouvelle combinaison de trois agents actifs cosmétiques sélectionnés pour l'effet synergique qu'ils procurent sur la synthèse de collagène de type I et de son précurseur le pro-collagène I dans des fibroblastes dermiques sénescents en culture. L'association de ces trois agents actifs est également efficace sur quatre autres marqueurs du vieillissement cutané après application topique sur explants de peau en survie.

La composition cosmétique de l'invention est particulièrement utile comme soin de la peau pour lutter contre la diminution de la fermeté de la peau, notamment comme agent cosmétique anti-âge restructurant et raffermissant de peaux âgées.

### Art Antérieur

Les fibroblastes sont les principales cellules du derme et sont spécialisées dans la synthèse des fibres de la matrice extra-cellulaire constituant le derme, notamment les fibres de collagène.

Le collagène est ainsi la principale protéine de soutien de la peau organisée en triple hélice à laquelle il confère sa résistance aux tensions et aux tractions.

Au cours du vieillissement, le défaut de collagène dans le derme est une des principales causes de la formation des rides. Avec l'âge, ce tissu de soutien de la peau s'affaiblit : les fibres de collagène se dégradent, leur renouvellement naturel est plus lent, le derme perd sa densité et ses propriétés mécaniques, il s'amincit, rides et ridules apparaissent, et les contours du visage s'affaissent.

Une étude a montré une importante diminution de la capacité de synthèse de collagène de type I par les fibroblastes de donneurs âgés (Dumas M et al, Mech Aging Dev; 1994, 73, 179-87).

Dans ce contexte, il apparaît primordial de relancer la synthèse de collagène *in vivo* afin de lutter efficacement contre le vieillissement cutané, en particulier au niveau du derme. Ceci peut en particulier être atteint en stimulant la synthèse du précurseur du collagène I, le pro-collagène I.

Les inventeurs ont trouvé une association de trois ingrédients agissant en synergie pour relancer la synthèse du collagène de type I et de son précurseur le pro-collagène I, dans des fibroblastes dermiques sénescents à un niveau très élevé.

Ces ingrédients actifs sont connus en tant qu'agents anti-âge, notamment pour leur activité vis-à-vis de la synthèse de collagène, néanmoins, il n'a jamais été montré jusqu'à présent qu'une telle association peut produire une synergie particulièrement remarquable quant à la synthèse de collagène de type I ou de son précurseur. La combinaison d'actifs présentant individuellement une activité anti-âge, ne présente pas nécessairement une activité anti-âge, comme le démontre les inventeurs dans les exemples qui suivent. En particulier, l'association de deux actifs connus comme anti-âge peut avoir une activité inférieure à la somme des activités des deux actifs pris séparément, et l'association de ces deux mêmes actifs à un troisième peut être dotée d'une activité anti-âge exceptionnelle. L'association d'actifs utilisée dans le cadre de l'invention est en outre également capable de stimuler d'autres marqueurs impliqués dans le vieillissement cutané comme les GAGs, l'élastine, mais aussi l'intégrine bêta-1.

Les glycosaminoglycanes (ou GAGs) sont des polysaccharides complexes, généralement sulfatés, retrouvés en abondance à la surface des cellules et dans la matrice extracellulaire. La polarité et les propriétés hydrophiles des GAGs les font implicitement participer à certaines fonctions biologiques.

Les GAGs neutres, observés au niveau des membranes basales telles que la jonction dermo-épidermique, autour des vaisseaux et des annexes cutanées sont un important réservoir de facteurs de croissance grâce à leur fonction de fixation de cations et de filtre ionique et macromoléculaire permettant de les concentrer aux abords de la cellule pour jouer leur rôle métabolique.

Les GAGs acides (essentiellement l'acide hyaluronique, un GAG non sulfaté), très impliqués dans le processus d'hydratation, grâce à leur aptitude à retenir l'eau, confèrent à la peau ses propriétés élastiques et de maintien du milieu extra- cellulaire. Ils sont principalement localisés dans le derme papillaire et dans les espaces inter-kératinocytaires des couches basales épidermiques. Le derme renferme la moitié de l'acide hyaluronique de l'organisme.

Ces deux types de GAGs constituent l'essentiel de la matrice extra-cellulaire. *In vivo,* les GAGs sont impliqués dans l'élasticité et l'hydratation de la peau. Le métabolisme et le renouvellement des GAGs, ainsi que la composition des chaînes de GAGs dépendent étroitement de l'état physiologique du tissu. Dans le processus de vieillissement, la synthèse des GAGs est modifiée, c'est le cas pour l'acide hyaluronique, qui représente environ 50% des GAGs cutanés, dont la synthèse est diminuée.

Il est donc utile de pouvoir stimuler la synthèse de ces GAGs au cours du vieillissement pour maintenir leurs effets positifs sur le maintien de la fermeté de la peau.

L'élastine confère aux fibres élastiques du derme leur élasticité et leur résilience et permet à la peau de reprendre sa position d'origine quand elle est pincée ou étirée. L'élastine est synthétisée et sécrétée dans l'espace extracellulaire par les fibroblastes et représente jusqu'à 90% des fibres élastiques. L'élastine et le collagène sont les principaux constituants de la matrice extracellulaire. La production totale d'élastine s'arrête autour de la puberté, après quoi, la quantité d'élastine disponible diminuera avec le temps.

L'exposition aux ultraviolets augmente la dégradation de l'élastine. Le changement le plus connu du photo-vieillissement, est la présence dans le derme réticulaire de l'élastose solaire qui résulte d'une accumulation de matériel constitué d'élastine anormale, et d'autres protéines de la matrice extracellulaire, comme la fibronectine ou la fibrilline. Le réseau de fibres élastiques montre alors des fibres souvent fragmentées, épaissies et non fonctionnelles.

L'élastine joue un rôle crucial dans le maintien d'une peau élastique, il est donc particulièrement utile de pouvoir en stimuler la synthèse pour prévenir ou ralentir les signes de vieillissement cutané.

Les intégrines sont des protéines transmembranaires dont l'une des extrémités interagit avec les structures situées à l'extérieur de la cellule, l'autre extrémité interagissant avec des constituants intracellulaires. La plupart d'entre elles se lient aux molécules de la matrice extracellulaire et aux microfilaments d'actine *via* un certain nombre de protéines de liaison qui s'associent à leur région intracellulaire. Les intégrines jouent un rôle très important dans la migration, la différenciation et la survie des cellules.

Une action sur ce marqueur est donc particulièrement recherchée pour une efficacité anti-âge.

### Description de l'invention

La composition de l'invention contient l'association d'un extrait d'algue brune, d'un extrait de levure et d'acide ascorbique ou un de ses esters. Elle a notamment un effet sur la synthèse de collagène de type I dans des fibroblastes dermiques vieillis en culture.

Il a été montré pour l'association de ces trois agents actifs cosmétiques, un effet synergique sur la synthèse de collagène de type I et de son précurseur le pro-collagène I : ces ingrédients ont des actions qui renforcent la capacité de synthèse de collagène de type I par des fibroblastes vieillis.

Les inventeurs ont trouvé que la composition de l'invention possède un fort potentiel de stimulation du collagène de type I et de son précurseur, bien que les concentrations des actifs soient faibles, ce qui permet de diminuer les quantités introduites dans les formules et le coût de revient des produits cosmétiques.

L'association d'actifs cosmétiques utilisée dans le cadre de l'invention présente également l'avantage d'être mise en formulation facilement par exemple par solubilisation en phase aqueuse, ce qui permet de produire une large gamme de produits cosmétiques anti-âge de soin et de maquillage de textures et de galéniques très variées allant de la crème au stick.

Ainsi, un premier objet de l'invention concerne une composition cosmétique comprenant un excipient cosmétiquement acceptable et l'association d'un extrait de l'algue brune *Laminaria digitata,* d'un hydrolysat de *Saccharomyces cerevisiae* et d'acide ascorbique ou un de ses esters.

L'association des trois ingrédients actifs représente au total de préférence de 0,01 à 1 % en poids par rapport au poids de la composition, les pourcentages étant exprimés en poids sec de l'association, de préférence de 0,05 à 0,6%, et de préférence encore de 0,1 à 0,3% en poids.

La concentration en extrait de l'algue brune *Laminaria digitata* dans la composition est de préférence comprise entre 0,005 et 0,02%, de préférence entre 0,007 et 0,015%, et de préférence encore entre 0,009 et 0,011% en poids, par rapport au poids de la composition, les pourcentages étant exprimés en poids sec d'extrait.

Dans un mode de réalisation, l'extrait d'algue brune est obtenu par broyage de l'algue fraîche, suivi d'une macération dans l'eau, d'une décantation et d'une filtration, chacune de ces étapes étant réalisée dans des conditions d'agitation, de température, de pH et de durée contrôlées. Un agent conservateur tel qu'un glycol peut être ajouté en fin de réaction.

Un tel extrait peut être mis en solution dans un solvant polaire, tel qu'un glycol, par exemple le butylène-glycol ou un mélange d'un glycol et d'eau.

Le nom INCI de l'extrait d'algue brune peut être *Laminaria digitata* extract (and) Butylene Glycol.

La concentration de l'hydrolysat de *Saccharomyces* dans la composition est de préférence comprise entre 0,001 et 0,05%, de préférence entre 0,003 et 0,01%, et de préférence encore entre 0,004% et 0,006% en poids, par rapport au poids de la composition, les pourcentages étant exprimés en poids sec de l'hydrolysat.

Dans un mode de réalisation, l'hydrolysat de *Saccharomyces* est obtenu par hydrolyse enzymatique d'une levure de boulanger *Saccharomyces cerevisiae.* Un hydrolysat au sens de l'invention n'est pas un simple ferment, car il vise à hydrolyser la levure et non à la cultiver. Les propriétés biologiques d'un hydrolysat ne sont donc pas nécessairement analogues à celles d'un ferment du même microorganisme.

L'hydrolysat de *Saccharomyces* peut être obtenu l'hydrolysat de *Saccharomyces* est obtenu par irradiation d'une suspension de la levure vivante par lumière UV et/ou chaleur, hydrolyse par une enzyme protéolytique, et recueil du filtrat.

Plus précisément, l'hydrolysat de *Saccharomyces* peut être obtenu par mise en culture de *Saccharomyces,* en particulier *Saccharomyces cerevisiae.* Les levures vivantes sont ensuite soumises dans leur milieu de culture à une irradiation par une source de lumière UV et/ou une source de chaleur. Cette irradiation met les levures en état de stress et provoque au bout d'un certain temps d'exposition des mécanismes de protection biochimiques complexes. Lorsque les mécanismes de protection sont terminés, on sépare les levures du milieu de culture et on procède à une hydrolyse par une enzyme protéolytique. Lorsque la réaction d'hydrolyse est terminée, l'enzyme est désactivée en la chauffant par exemple à 80°C pendant une heure. Les parois cellulaires sont ensuite éliminées par filtration. La teneur en matière sèche de l'extrait peut être ajustée par dilution dans l'eau, et atteindre par exemple 25% en poids.

Un hydrolysat de *Saccharomyces cerevisiae* peut être obtenu par le procédé comprenant la série d'étapes suivantes :
- mise en suspension de la levure vivante dans un milieu de culture approprié ;
- irradiation du milieu de culture par lumière UV et/ou apport de chaleur pour mettre les cellules en situation de stress ;
- retrait du milieu nourrissant et lyse des cellules de la levure par une enzyme protéolytique.
- désactivation de l'enzyme; et
- filtration pour éliminer les parois cellulaires, et recueil du filtrat.

Un exemple de ce procédé est donné dans le document US 2002/0192765.

Le nom INCI de l'hydrolysat de *Saccharomyces* peut être Water (and) *Saccharomyces* lysate extract.

La concentration en acide ascorbique ou un de ses esters peut être comprise entre 0,05 et 0,5%, de préférence entre 0,1 et 0,3%, et de préférence encore entre 0,15 et 0,25% en poids, par rapport au poids de la composition.

Un ester d'acide ascorbique peut être le 2-ascorbyl-glucoside de formule (I) :

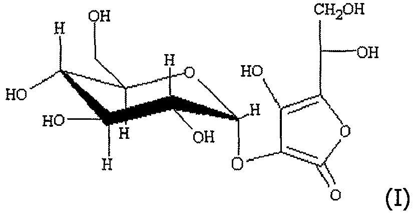

La concentration en 2-ascorbyl-glucoside est de préférence comprise entre 0,05 et 0,5% en poids par rapport au poids de la composition.

Le ratio massique entre l'extrait de l'algue brune *Laminaria digitata* et l'hydrolysat de *Saccharomyces* est avantageusement compris entre 1/1 et 5/1, de préférence entre 1/1 et 3/1, notamment de l'ordre de 2/1.

Le ratio massique entre l'acide ascorbique ou un de ses esters et l'hydrolysat de *Saccharomyces* est avantageusement compris entre 10/1 et 100/1, de préférence entre 30/1 et 50/1, notamment de l'ordre de 40/1.

Selon un aspect de l'invention, la composition de l'invention est une composition de soin cosmétique, par exemple une composition de soin anti-âge, comprenant l'association de l'invention en tant qu'agent actif anti-âge et éventuellement au moins un autre agent actif cosmétique.

Ledit agent actif anti-âge est constitué de l'association d'un extrait de l'algue brune *Laminaria digitata,* d'un hydrolysat de *Saccharomyces,* et d'acide ascorbique ou un de ses esters.

Dans un mode de réalisation, l'actif anti-âge constitué de l'association d'un extrait de l'algue brune *Laminaria digitata,* d'un hydrolysat de *Saccharomyces,* et de l'acide ascorbique ou un de ses esters représente entre 0,01 et 1% en poids du poids de la composition, par exemple de l'ordre de 0,2 % en poids.

La composition cosmétique peut en outre également comprendre un ou plusieurs autres agents actifs cosmétiques, choisis notamment parmi ceux ayant une effet anti-âge et/ou une activité anti-radicalaire et/ou un effet dépigmentant ou éclaircissant de la peau et/ou un effet amincissant et/ou un effet hydratant et/ou un effet calmant, apaisant ou relaxant et/ou un effet de stimulation de la microcirculation cutanée et/ou un effet sébo-régulateur et/ou un effet nettoyant ou purifiant de la peau.

La composition est de préférence dépourvue d'un autre agent actif anti-âge stimulant la synthèse du collagène, que celui décrit précédemment constitué de l'association d'un extrait de l'algue brune *Laminaria digitata,* d'un hydrolysat de *Saccharomyces,* et d'acide ascorbique ou un de ses esters.

L'action de l'actif anti-âge de l'invention peut être avantageusement complétée par l'action d'autres agents anti-âge agissant sur une autre cible du vieillissement.

En particulier, la composition cosmétique peut comprendre un ou plusieurs autres agents actifs cosmétiques choisis notamment parmi :
- des agents stimulants la fermeté de la peau, tels que des peptides stimulant la synthèse d'autres types de collagène que celui de type I, en particulier le collagène de type III, ou VII, un extrait de *Centella asiatica,* l'acide madécassique, l'acide asiatique, le madécassoside, un extrait d'avoine, un extrait de *Bertholletia excelsa* , un hydrolyzat de protéines, des peptides de soja, un extrait de *Potentilla erecta,* un extrait de *Siegesbeckia orientalis,* des ginsenosides ou notoginsenosides.
- des inhibiteurs d'élastase tels que des extraits *d'Aspergillus fumigatus, Momordica charantia, Cucurbita maxima ;*
- des agents stimulant la synthèse de dermatopontine, comme un extrait d'ambre ;
- des agents resserrant les pores tels que des extraits de plantes astringentes, par exemple un extrait *d'Hamamelis sp. ;*
- des filtres physiques ou chimiques protégeant des radiations UVA et UVB, tels que la benzophénone, le 4-butyl méthoxydibenzoylméthane, l'octocrylène, l'éthylhexyl méthoxycinnamate, l'éthylhexyl salicylate, l'acide phénylbenzymidazole sulfonique, l'homosalate, seuls ou en association avec des oxydes de titane ;
- des agents destinés à combattre les désordres de pigmentation, notamment ceux liés au vieillissement cutané, tels que l'acide kojique, les extraits de racine de réglisse ou de murier, l'arbutine, le pantothénosulfonate de calcium, la boldine, la diacétylboldine, des extraits de lys, en particulier de bulbe ;
- des agents anti-radicalaires ou anti-inflammatoires tels qu'un extrait d'*Artemisia capillaris,* un extrait de *Sanguisorba officinalis,* le resvératrol et ses dérivés, le curcuma ou la curcumine ou la tétrahydrocurcumine, des polyphénols extraits de pépins de raisin, de la vitamine E et ses dérivés, en particulier ses dérivés phosphate, l'ergothioneine ou ses dérivés, l'idébénone ;
- l'aspartate de magnésium ou l'adénosine pour améliorer l'action antirides ;
- du D-xylose pour améliorer le « plumping effect » pour une peau rebondie;
et l'un quelconque de leurs mélanges.

On pourra compléter l'action de l'association des trois principes actifs décrite précédemment par un actif anti-âge agissant sur la synthèse des lipides pour un effet relipidant améliorant la fonction barrière ou un actif anti-âge modérant les micro-inflammations impliquées dans le processus de vieillissement.

Avantageusement, la composition comprend en outre au moins un excipient cosmétiquement acceptable pouvant être choisi parmi des pigments, des colorants, des polymères, des agents tensioactifs, des agents de rhéologie, des parfums, des électrolytes, des ajusteurs de pH, des conservateurs, et leurs mélanges.

La composition cosmétique peut être par exemple un sérum, une lotion, une crème (émulsion huile-dans-eau) ou bien encore un hydrogel, de préférence un masque, ou encore se présenter sous la forme d'un stick ou d'un patch.

Dans un mode de réalisation particulier, la composition se présente sous la forme d'un sérum anti-âge comprenant plus de 85% en poids d'eau, de préférence plus de 95% en poids d'eau.

Dans un autre mode de réalisation, la composition se présente sous la forme d'une émulsion huile-dans-eau comprenant jusqu'à 40% en poids, de préférence jusqu'à 30% en poids, d'une phase grasse par rapport au poids total de la composition.

Un deuxième objet de l'invention porte sur une méthode de soin cosmétique comprenant l'application topique i) de la composition décrite précédemment, ou ii) de l'association d'un extrait de l'algue brune *Laminaria digitata,* d'un hydrolysat de *Saccharomyces* et d'acide ascorbique ou un de ses esters, sur une peau présentant des signes de vieillissement intrinsèque et/ou extrinsèque, tels que les rides, les ridules, une perte d'élasticité, le relâchement ou le flétrissement.

La composition ou l'association des trois composés est appliquée sur la peau d'un individu ayant de préférence plus de 30 ans, plus de 35 ans et de préférence encore plus de 40 ans.

Selon un mode de réalisation, la composition contenant
- entre 0,005 et 0,02%, de préférence entre 0,007 et 0,015%, et de préférence encore entre 0,009 et 0,011%, en poids de l'extrait de l'algue brune *Laminaria digitata* par rapport au poids de la composition,
- entre 0,001 et 0,05%, de préférence entre 0,003 et 0,01%, et de préférence encore entre 0,004% et 0,006%, en poids de l'hydrolysat de *Saccharomyces* par rapport au poids de la composition.
- entre 0,05 et 0,5%, de préférence entre 0,1 et 0,3%, et de préférence encore entre 0,15 et 0,25%, en poids d'acide ascorbique ou un de ses esters par rapport au poids de la composition,
est appliquée sur la peau à raison de 1 à 5 mg/cm², de préférence de l'ordre de 2 mg/cm².

Enfin, un troisième objet de l'invention concerne l'utilisation de l'association d'un extrait de l'algue brune *Laminaria digitata,* d'un hydrolysat de *Saccharomyces* et de l'acide ascorbique ou un de ses esters, pour simultanément i) augmenter la synthèse de collagène I ou de son précurseur le pro-collagène I et ii) avoir au moins un autre effet choisi parmi l'expression des GAG, la synthèse de l'élastine et l'expression des intégrines dans la peau d'un individu.

L'invention concerne également la composition cosmétique comprenant un excipient cosmétiquement acceptable et l'association d'un extrait de l'algue brune *Laminaria digitata,* d'un hydrolysat de *Saccharomyces* et de l'acide ascorbique ou un de ses esters, pour son utilisation dans le traitement ou la prévention du vieillissement de la peau, en particulier du derme de la peau, de préférence chez un individu de plus de 30 ans.

Elle permet notamment de diminuer l'intensité des rides et des ridules et de restaurer la structure de la matrice extra-cellulaire constituant le derme. Elle provoque un effet tenseur de la surface de la peau en restaurant le réseau des fibres de collagène. Cet effet tenseur est perceptible au niveau des contours du visage.

L'invention sera illustrée plus en détail par les exemples de réalisation suivants.

### EXEMPLE 1 : essais sur fibroblastes

On étudie l'influence des extraits constituant l'association de l'invention sur la synthèse de pro-collagène I par des fibroblastes humains normaux (FHN). Le pro-collagène est la forme précurseur du collagène sécrété dans le milieu extra cellulaire où les extrémités sont coupées pour aboutir à la sub-unité de collagène. L'association de ces molécules de collagène va ensuite créer les fibres de collagène.

### Matériels et méthodes

### 1. Modèle cellulaire

- Type : Fibroblastes humains normaux (FHN, donneur de 37 ans), vieillis par passages en culture (20^{ème} passage).
- Conditions de culture : 37°C, 5% CO₂
- Milieu de culture : (Invitrogen 21090-22) complémenté avec L-Glutamine 2 mM, Penicilline 50 UI/ml - Streptomycine 50 µg/ml, SVF 10% (Biowest S1810).
- Milieu d'essai : milieu de culture à 1% en sérum de veau foetal (SVF).

Les actifs sont mis en solution dans le milieu de culture aux concentrations précisées dans le tableau suivant.

| Nom Commercial/ Fournisseur | Nom INCI | Doses testées Sur fibroblastes en culture |
|---|---|---|
| Raffermine®/Silab | Hydrolyzed Soy Flour | 0,035 % |
| Seanergilium® BG/BASF | *Laminaria digitata* extract (and) Butylene Glycol | 0,02 % |
| Biodynes® TRF® improved 25/ARCH | Water & *Saccharomyces* Lysate Extract | 0,005 % |
| Vitamine C | Ascorbic acid | 0,002% |

### 2. Culture et traitements

La synthèse du Pro-collagène de type I par ces cellules a été évaluée par la technique ELISA à partir des surnageants de culture de fibroblastes vieillis.

Les FHN sont mis en culture en microplaque 96 puits à raison de 7500 cellules/puits pendant 24 heures. A 80% de confluence, le milieu est remplacé par le milieu d'essai contenant les agents actifs à tester ou les références. Les cellules sont incubées pendant 72 heures. Les surnageants de culture sont ensuite récupérés et stockés à -20°C en attente de dosage. Un dosage des protéines totales (BCA) est réalisé sur le tapis cellulaire.

Le dosage du Pro-collagène I est réalisé à l'aide d'un kit ELISA (Takara).

Les quantités de pro-collagène I sont exprimées en ng/mg de protéine.

### Résultats

Dans chaque expérience, les séries de valeurs témoins et de valeurs traitées sont comparées à l'aide d'un test de Student (séries non appariées). Les différences sont significatives pour les valeurs de p < 0,05.

### 1, Effets des actifs seuls.

Les résultats obtenus pour chacun des actifs testés seuls sont présentés dans le tableau ci-dessous.

| n° | Actifs | Pro Coll. I (ng/mg de prot) | Variation | Significativité vs témoin |
|---|---|---|---|---|
| | Témoin non traité | 1439 ± 161 | - | |
| 1 | Raffermine® | 3755 ± 56 | + 154% | S |
| 2 | Seanergillum® | 2604 ± 203 | + 81% | S |
| 3 | Biodynes® | 2469 ± 214 | + 72% | S |
| 4 | Vitamine C | 2431 ± 55 | + 67% | S |

| | | | | |
|---|---|---|---|---|
| S = significatif | | | | |

Ces résultats sont utilisés ci-dessous pour comparer l'effet d'associations de deux ou trois de ces agents actifs, par rapport à l'effet théorique attendu calculé à partir des valeurs obtenues pour chaque actif individuellement.

### 2. Effets de l'association de deux agents actifs dont un à base d'un extrait de Saccharomyses

Le tableau ci-dessous regroupe les résultats obtenus pour les différentes associations d'actifs avec l'extrait de levure (*Saccharomyces*):

| n° | Actif associé à Biodynes® | Pro Coll. I (ng/mg de prot) | Variation | Significativité vs témoin |
|---|---|---|---|---|
| | Témoin non traité | 1427 ± 154 | - | |
| 5 | Raffermine® + Biodynes® | 3996 ± 227 | + 180% | S |
| 6 | Seanergillum® + Biodynes® | 2321 ± 172 | + 63% | S |
| 7 | Vitamine C+ Biodynes® | 2780 ± 390 | + 95% | S |

Quand on compare le résultat obtenu pour chacune des associations ci-dessus avec l'effet théorique attendu calculé d'après les valeurs obtenues au paragraphe 1, on note que l'association à base d'un extrait de levure ne procure aucun effet de synergie mesurable. L'activité mesurée pour une association est toujours inférieure à celle calculée.

| | Activité mesurée | Activité calculée |
|---|---|---|
| Raffermine®+Biodynes® | +180% | + 226% |
| Seanergillum®+Biodynes® | + 63% | + 153% |
| Vitamine C+ Biodynes® | + 95% | + 139% |

### 3. Effets d'une association de deux agents actifs dont un à base d'acide ascorbique.

Les résultats obtenus pour différentes associations à base de 2-ascorbyl-glucoside sont indiqués dans le tableau ci-dessous.

| | test | Moyenne (ng/mg de prot) | Variation | Significativité vs témoin |
|---|---|---|---|---|
| | Témoin non traité | 1033 ± 174 | | |
| 8 | Raffermine® + Vitamine C | 4957 ± 907 | + 380% | S |
| 9 | Seanergillum® + Vitamine C | 4822 ± 740 | + 367% | S |
| 10 | Biodynes® + Vitamine C | 2016 ± 83 | + 95% | S |

Pour deux des trois associations, le 2-ascorbyl-glucoside potentialise de façon très importante la synthèse de Pro-collagène I. Les résultats du tableau ci-dessous confirment également que l'extrait de levure ne semble pas être un bon candidat dans une association (ici avec l'acide ascorbique) pour stimuler la synthèse de pro-collagène I.

| | Activité mesurée | Activité calculée |
|---|---|---|
| Raffermine® + Vitamine C | + 380% | + 221% |
| Seanergillum® + Vitamine C | + 367% | + 148% |
| Biodynes® + Vitamine C | + 95% | + 139% |

### 4. Effets d'une association de trois agents actifs dont un à base d'acide ascorbique et un à base d'extrait de levure.

Les résultats obtenus pour ces différentes associations de trois agents actifs sont regroupés dans le tableau ci-dessous :

| n° | Test | Moyenne (ng/mg de prot) | Variation | Significativité vs témoin |
|---|---|---|---|---|
| | Témoin | 258 ± 22 | - | |
| 11 | Raffermine® + Biodynes® + Vitamine C | 996 ± 37 | + 286% | S |
| 12 | Seanergillum®+ Biodynes® + Vitamine C | 1567 ± 71 | + 507% | S |

On compare ci-dessous l'activité mesurée pour l'association des trois actifs avec celle théorique calculée à partir de l'activité de chacun des actifs pris séparément.

### Raffermine®+Biodynes®+Vitamine C

| n° | Activité mesurée | Activité calculée |
|---|---|---|
| 10 | +286% | |
| 1+3+4 | | + 293% |

Pour cette association, on constate que l'activité mesurée est légèrement inférieure à l'activité attendue calculée à partir des tests précédents. On n'observe aucune synergie susceptible de retenir une telle association pour le traitement du vieillissement cutané.

### Seanergillum®+Biodynes®+Vitamine C

| n° | Activité mesurée | Activité calculée |
|---|---|---|
| 12 | +507% | |
| 2+3+4 | | + 220% |

L'association présente un effet de synergie sur la synthèse du pro-collagène I. En effet, on constate que dans le cas de l'association comprenant l'extrait de l'algue brune *Laminaria digitata,* un extrait de levure et l'acide ascorbique, l'activité mesurée est très nettement supérieure à celle attendue.

On observe également de façon très surprenante que dans le cas de l'association de l'invention, et contrairement à l'association précédente, on ne mesure pas d'effet négatif lié à la présence de l'extrait de levure, et qu'au contraire l'effet positif mesuré sur la synthèse de pro-collagène I est bien supérieur à celui mesuré ou calculé pour toutes les associations précédemment testées.

Cette association est donc particulièrement intéressante pour lutter contre l'apparition des signes de vieillissement cutanée, sous la forme de compositions cosmétiques.

### EXEMPLE 2 : Essais sur modèle de peau entière maintenue en survie

Cette étude a pour but d'explorer l'activité anti-âge de formulations cosmétiques sur les structures épidermiques et dermiques d'explants de peau humaine ex *vivo.*

Cette activité est évaluée par une visualisation des glycosaminoglycanes (ou GAGs), Immunomarquage du collagène I, Immunomarquage de l'Elastine, et Immunomarquage de l'Intégrine bêta-1.

On prépare des explants de 10 mm de diamètre à partir d'une plastie adbominale d'une femme âgée de 50 ans. Les explants ont été mis en survie en milieu BEM (BIO-EC's Explants Médium) à 37°C en atmosphère humide, enrichie de 5 % de CO2.

### Conditions de l'étude :

On teste différentes compositions cosmétiques de l'invention et des compositions comparatives. Les compositions cosmétiques de l'invention comprennent l'association de l'invention constituée d'un extrait de l'algue brune *Laminaria digitata,* d'un extrait de levure et d'un ester de l'acide ascorbique,

Chaque composition cosmétique comprend ainsi :
- 0,01% en poids sec de Seanergilium® BG/BASF (extrait de *Laminaria digitata,*
- 0,005% en poids sec de Biodynes® TRF® improved 25/ARCH (extrait de Saccharomyces) et
- 0,2% en poids de 2-ascorbyl-glucoside, un ester de l'acide ascorbique.

Ces compositions cosmétiques ont un taux de glycols constant à 7% en poids et se distinguent les unes des autres par une proportion variable en phase grasse.

Les textures testées sont les suivantes :
- une émulsion huile-dans-eau (H/E) à 20% en poids de phase grasse, notée E₂₀ comprenant 20% de phase grasse,
- une émulsion H/E placebo, exempte de l'association de l'invention, à 20% de phase grasse, notée Emulsion Placebo P₂₀,
- une émulsion H/E à 10% en poids de phase grasse, notée E₁₀
- un sérum gélifié comprenant 1,5% en poids de phase grasse, noté S

Pour chaque composition cosmétique à tester, on réalise cinq applications successives d'une dose de 2 mg par cm² sur un explant de peau à J0, J2, J4, J6 et J8. On prépare ainsi au moins trois explants, par exemple 6, sur lesquels on réalise la mesure de chaque marqueur à J9 ou à J10. Pour chaque marqueur, on réalise la moyenne de tous les explants préparés.

L'expression des GAGs est évaluée par coloration au trichrome des coupes d'explants réalisées dans de la paraffine. La coloration est ensuite évaluée au microscope par analyse d'image.

L'élastine, le collagène I et l'intégrine bêta-1 sont quantifiées par immunomarquage, et analyse microscopique.

Pour chaque marqueur testé, les résultats obtenus sur les explants de peau en survie sont exprimés en pourcentages de variation par rapport au témoin non traité, uniquement pour les résultats statistiquement significatifs.

Les variations non significatives sont notées NS.

Le tableau ci-dessous montre les variations d'activité, en % par rapport à un témoin non traité, pour une émulsion à 20% en poids de phase grasse comprenant ou non l'association de l'invention :

| Formules/Témoin NT J10 | GAGs | Collagène I | Intégrine bêta-1 |
|---|---|---|---|
| Emulsion Placebo P₂₀ | NS | NS | NS |
| Emulsion E₂₀ | + 51% | + 43% | + 50% |

On montre que les compositions cosmétiques de l'invention présentent une activité significative sur un grand nombre de marqueurs que pour l'émulsion placebo (Emulsion Placebo P₂₀).

Le test est également mené à l'aide d'une émulsion comprenant une phase grasse en plus faible proportion.

On exprime dans le tableau ci-dessous les variations d'activité en pourcentage par rapport à un témoin non traité pour chacun des marqueurs testés :

| Formules/Témoin NT J9 | GAGs | Collagène I | Elastine |
|---|---|---|---|
| Sérum gélifié S | + 60 % | + 126 % | + 33% |
| Emulsion E₁₀ | + 44% | + 140% | NS |

L'association montre ainsi tout son intérêt comme agent actif anti-âge dans des produits variés destinés au soin des peaux âgées ou présentant des signes de vieillissement cutané.

Les résultats obtenus à la fois pour l'association elle-même ou pour des compositions cosmétiques comprenant l'association de l'invention, démontre l'efficacité de l'association de l'invention dans des compositions cosmétiques qui présentent des textures cosmétiques différentes, pour le soin ou le maquillage de la peau.

L'association de l'invention stimule significativement différents marqueurs fortement impliqués dans le vieillissement cutané comme les GAGs, l'élastine ou l'intégrine bêta-1.

Parmi les compositions pour la peau, le sérum gélifié, qui présente la plus faible proportion de phase grasse, montre une efficacité importante sur le plus grand nombre de marqueurs testés.

L'association de l'invention pour laquelle il a été montré un effet de synergie vis-à-vis de la synthèse de pro-collagène I, produit également un effet intéressant vis-à-vis d'autres marqueurs du vieillissement, notamment le collagène I.

Celle-ci présente donc un intérêt de premier ordre pour une utilisation dans des compositions anti-âge, ou des méthodes de soin ou de maquillage visant à lutter contre l'apparition de signes de vieillissement cutané ou à en ralentir l'évolution.

## Revendications

1. Composition cosmétique comprenant au moins un excipient cosmétiquement acceptable et un mélange comprenant
- un extrait de l'algue brune *Laminaria digitata,*
- un hydrolysat de *Saccharomyces* et
- l'acide ascorbique ou un de ses esters,
en une quantité allant de 0,01 à 1 % en poids par rapport au poids de la composition, les pourcentages étant exprimés en poids sec du mélange.

2. Composition selon la revendication 1, **caractérisée en ce que** la concentration en poids de l'extrait de l'algue brune *Laminaria digitata* est comprise entre 0,005 et 0,02% en poids, par rapport au poids de la composition, les pourcentages étant exprimés en poids sec d'extrait.

3. Composition selon les revendications 1 ou 2, **caractérisée en ce que** la concentration en poids de l'hydrolysat de *Saccharomyces* est comprise entre 0,001 et 0,05% par rapport au poids de la composition, les pourcentages étant exprimés en poids sec d'hydrolysat.

4. Composition selon l'une quelconque des revendications précédentes, **caractérisée en ce que** la concentration en acide ascorbique ou un de ses esters est comprise entre 0,05 et 0,5% en poids, par rapport au poids de la composition.

5. Composition selon l'une quelconque des revendications précédentes, **caractérisée en ce que** l'extrait de l'algue brune *Laminaria digitata* est obtenu par broyage de l'algue fraîche, suivi d'une macération dans l'eau, d'une décantation et d'une filtration.

6. Composition selon l'une quelconque des revendications précédentes, **caractérisée en ce que** l'hydrolysat de *Saccharomyces* est obtenu par irradiation d'une suspension de la levure vivante par lumière UV et/ou chaleur, hydrolyse par une enzyme protéolytique, et recueil du filtrat.

7. Composition selon l'une quelconque des revendications précédentes, **caractérisée en ce que** le ratio massique entre l'extrait de l'algue brune *Laminaria digitata* et l'hydrolysat de *Saccharomyces* est compris entre 1/1 et 5/1.

8. Composition selon l'une quelconque des revendications précédentes, **caractérisée en ce que** le ratio massique entre l'acide ascorbique ou un de ses esters et l'hydrolysat de *Saccharomyces* est compris entre 10/1 et 100/1.

9. Composition selon l'une quelconque des revendications précédentes, **caractérisée en ce qu'**elle se présente sous la forme d'un sérum anti-âge comprenant plus de 85% en poids d'eau ou d'une émulsion huile-dans-eau comprenant jusqu'à 40% en poids d'une phase grasse par rapport au poids total de la composition.

10. Méthode de soin cosmétique comprenant l'application topique i) de la composition selon l'une des revendications précédentes, ou ii) de l'association d'un extrait de l'algue brune *Laminaria digitata,* d'un hydrolysat de *Saccharomyces* et d'acide ascorbique ou un de ses esters, sur une peau présentant des signes de vieillissement intrinsèque et/ou extrinsèque, tels que les rides, les ridules, le relâchement ou le flétrissement.

11. Méthode de soin cosmétique selon la revendication 10, **caractérisée en ce que** la composition ou l'association est appliquée sur la peau d'un individu ayant plus de 30 ans.

12. Méthode de soin cosmétique selon l'une des revendications 10 à 11, **caractérisée en ce que** la composition contenant
- entre 0,005 et 0,02% en poids de l'extrait de l'algue brune *Laminaria digitata* par rapport au poids de la composition,
- entre 0,001 et 0,05% en poids de l'hydrolysat de *Saccharomyces* par rapport au poids de la composition.
- entre 0,05 et 0,5% en poids d'acide ascorbique ou un de ses esters par rapport au poids de la composition,
est appliquée sur la peau à raison de 1 à 5 mg/cm².

13. Utilisation de l'association d'un extrait de l'algue brune *Laminaria digitata,* d'un hydrolysat de *Saccharomyces* et d'acide ascorbique ou un de ses esters, pour simultanément i) augmenter la synthèse de collagène I ou de son précurseur le pro-collagène I et ii) avoir au moins un autre effet choisi parmi l'expression des GAG, la synthèse de l'élastine et l'expression des intégrines dans la peau d'un individu.

14. Composition cosmétique comprenant au moins un excipient cosmétiquement acceptable et l'association d'un extrait de l'algue brune *Laminaria digitata,* d'un hydrolysat de *Saccharomyces* et d'acide ascorbique ou un de ses esters, pour son utilisation dans le traitement ou la prévention du vieillissement de la peau.

## Patentansprüche

1. Kosmetische Zusammensetzung, umfassend mindestens einen kosmetisch unbedenklichen Hilfsstoff und eine Mischung, umfassend
- einen Extrakt aus Braunalgen *Laminaria digitata,*
- ein *Saccharomyces*-Hydrolysat und
- Ascorbinsäure oder einen ihrer Ester,
in einer Menge im Bereich von 0,01 bis 1 Gew. -% bezogen auf das Gewicht der Zusammensetzung, wobei die Prozentsätze in Trockengewicht der Mischung ausgedrückt werden.

2. Zusammensetzung gemäß Anspruch 1, **dadurch gekennzeichnet, dass** die Gewichtskonzentration des Extraktes der Braunalge *Laminaria digitata* zwischen 0,005 und 0,02 Gew. -% bezogen auf das Gewicht der Zusammensetzung liegt, wobei die Prozentsätze in Trockengewicht des Extraktes ausgedrückt sind.

3. Zusammensetzung gemäß Anspruch 1 oder 2, **dadurch gekennzeichnet, dass** die Gewichtskonzentration von *Saccharomyces*-Hydrolysat zwischen 0,001 und 0,05 % bezogen auf das Gewicht der Zusammensetzung beträgt, wobei die Prozentsätze in Trockengewicht von Hydrolysat ausgedrückt sind.

4. Zusammensetzung gemäß einem der vorangehenden Ansprüche, **dadurch gekennzeichnet, dass** die Konzentration der Ascorbinsäure oder eines ihrer Ester zwischen 0,05 und 0,5 Gew. -% bezogen auf das Gewicht der Zusammensetzung liegt.

5. Zusammensetzung gemäß einem der vorangehenden Ansprüche, **dadurch gekennzeichnet, dass** der Extrakt aus Braunalgen *Laminaria digitata* durch Zerkleinern der frischen Algen gewonnen wird, gefolgt von Einweichen in Wasser, Dekantieren und Filtrieren.

6. Zusammensetzung gemäß einem der vorangehenden Ansprüche, **dadurch gekennzeichnet, dass** *Saccharomyces-Hydrolysat* durch Bestrahlen einer Suspension der lebenden Hefe mit UV-Licht und/oder Wärme, Hydrolyse durch ein proteolytisches Enzym und Sammeln des Filtrats erhalten wird.

7. Zusammensetzung gemäß einem der vorangehenden Ansprüche, **dadurch gekennzeichnet, dass** das Massenverhältnis zwischen dem Extrakt aus Braunalgen *Laminaria digitata* und dem *Saccharomyces*-Hydrolysat zwischen 1/1 und 5/1 beträgt.

8. Zusammensetzung gemäß einem der vorangehenden Ansprüche, **dadurch gekennzeichnet, dass** das Massenverhältnis zwischen Ascorbinsäure oder einem ihrer Ester und dem *Saccharomyces*-Hydrolysat zwischen 10/1 und 100/1 beträgt.

9. Zusammensetzung gemäß einem der vorangehenden Ansprüche, **dadurch gekennzeichnet, dass** sie in Form eines Anti-Aging-Serums vorliegt, das mehr als 85 Gew. -% Wasser oder einer ÖI-in-Wasser-Emulsion enthält, die bis zu 40 Gew. -% einer Fettphase bezogen auf das Gesamtgewicht der Zusammensetzung umfasst.

10. Kosmetisches Pflegeverfahren, umfassend das topische Auftragen von (i) der Zusammensetzung gemäß einem der vorangehenden Ansprüche oder (ii) der Kombination eines Extraktes aus Braunalgen *Laminaria digitata,* eines *Saccharomyces*-Hydrolysats und Ascorbinsäure oder eines ihrer Ester auf eine Haut, die Anzeichen von intrinsischer und/oder extrinsischer Alterung aufweist, wie Falten, Fältchen, Durchhängen oder Welken.

11. Kosmetisches Pflegeverfahren gemäß Anspruch 10, **dadurch gekennzeichnet, dass** die Zusammensetzung oder Kombination auf die Haut einer Person über 30 Jahre aufgetragen wird.

12. Kosmetisches Pflegeverfahren gemäß einem der Ansprüche 10 bis 11, **dadurch gekennzeichnet, dass** die Zusammensetzung, die enthält
- zwischen 0,005 und 0,02 Gew. -% des Extraktes aus Braunalgen *Laminaria digitata* bezogen auf das Gewicht der Zusammensetzung,
- zwischen 0,001 und 0,05 Gew. -% *Saccharomyces*-Hydrolysat bezogen auf das Gewicht der Zusammensetzung,
- zwischen 0,05 und 0,5 Gew. -% Ascorbinsäure oder einem ihrer Ester bezogen auf das Gewicht der Zusammensetzung,
in einer Menge von 1 bis 5 mg/cm² auf die Haut aufgetragen wird.

13. Verwendung der Kombination eines Extraktes aus Braunalgen *Laminaria digitata, Saccharomyces*-Hydrolysat und Ascorbinsäure oder eines ihrer Ester, um gleichzeitig (i) die Synthese von Kollagen I oder seines Vorläufers pro-Kollagen I zu erhöhen und (ii) mindestens eine weitere Wirkung aufzuweisen, auszuwählen aus der Expression von GAGs, der Elastinsynthese und der Expression von Integrinen in der Haut eines Individuums.

14. Kosmetische Zusammensetzung, umfassend mindestens einen kosmetisch unbedenklichen Hilfsstoff und die Kombination eines Extraktes aus Braunalgen *Laminaria digitata,* eines *Saccharomyces*-Hydrolysats und Ascorbinsäure oder eines ihrer Ester zur Verwendung bei der Behandlung oder Vorbeugung von Hautalterung.

## Claims

1. A cosmetic composition comprising at least one cosmetically acceptable excipient and the mixture comprising
- an extract of the brown alga *Laminaria digitata,*
- a *Saccharomyces* hydrolysate and
- ascorbic acid and one of its esters,
in an amount ranging from 0.01 to 1% by weight, with respect to the weight of the composition, the percentage being expressed by dry weight of the mixture.

2. The composition as claimed in claim 1, **characterized in that** the concentration by weight of the extract of the brown alga *Laminaria digitata* is between 0.005 and 0.02% by weight, with respect to the weight of the composition, the percentage being expressed by weight of extract.

3. The composition as claimed in claim 1 or claim 2, **characterized in that** the concentration by weight of the *Saccharomyces* hydrolysate is between 0.001 and 0.05%, with respect to the weight of the composition, the percentages being expressed by dry weight of hydrolysate.

4. The composition as claimed in any one of the preceding claims, **characterized in that** the concentration of ascorbic acid or one of its esters is between 0.05 and 0.5% by weight, with respect to the weight of the composition.

5. The composition as claimed in any one of the preceding claims, **characterized in that** the extract of the brown alga *Laminaria digitata* is obtained by grinding the fresh alga, followed by the steps of maturation in water, separation, settling and filtration.

6. The composition as claimed in any one of the preceding claims, **characterized in that** the *Saccharomyces* hydrolysate is obtained by irradiation of a suspension of the living yeast with UV light and/or heat, hydrolysis by a proteolytic enzyme and collection of the filtrate.

7. The composition as claimed in any one of the preceding claims, **characterized in that** the ratio by weight of the extract of the brown alga *Laminaria digitata* to the *Saccharomyces* hydrolysate is between 1/1 and 5/1.

8. The composition as claimed in any one of the preceding claims, **characterized in that** the ratio by weight of the ascorbic acid or one of its esters to the *Saccharomyces* hydrolysate is between 10/1 and 100/1.

9. The composition as claimed in any one of the preceding claims, **characterized in that** it is provided in the form of an anti-aging serum comprising more than 85% by weight of water or of an oil-in-water emulsion comprising up to 40% by weight of a fatty phase, with respect to the total weight of the composition.

10. A cosmetic care method comprising the topical application i) of the composition as claimed in one of the preceding claims or ii) of the combination of an extract of the brown alga *Laminaria digitata,* of a *Saccharomyces* hydrolysate and an ascorbic acid or one of its esters to skin exhibiting signs of intrinsic and/or extrinsic aging, such as wrinkles, fine lines, slackening or withering.

11. The cosmetic care method as claimed in claim 10, **characterized in that** the composition or the combination is applied to the skin of an individual aged more than 30 years.

12. The cosmetic care method as claimed in either of claims 10 and 11, **characterized in that** the composition containing
- between 0.005 and 0.02% by weight of the extract of the brown alga *Laminaria digitata,* with respect to the weight of the composition,
- between 0.001 and 0.05% by weight of the *Saccharomyces* hydrolysate, with respect to the weight of the composition,
- between 0.05 and 0.5% by weight of ascorbic acid or one of its esters, with respect to the weight of the composition,
is applied to the skin in a proportion of 1 to 5 mg/cm².

13. The use of the combination of an extract of the brown alga *Laminaria digitata,* of a *Saccharomyces* hydrolysate and of ascorbic acid or one of its esters for simultaneously i) increasing the synthesis of collagen I or of its precursor, procollagen I, and ii) having at least one other effect chosen from the expression of GAGs, the synthesis of elastin and the expression of integrins in the skin of an individual.

14. A cosmetic composition comprising at least one cosmetically acceptable excipient and the combination of an extract of the brown alga *Laminaria digitata,* of a *Saccharomyces* hydrolysate and of ascorbic acid or one of its esters for its use in the treatment or the prevention of aging of the skin.
